# EUROPEAN PATENT SPECIFICATION

(11) **EP 3 475 664 B1**
(45) Date of publication and mention of the grant of the patent: **02.12.2020**
(21) Application number: 17745453.5
(22) Date of filing: 27.06.2017
(51) Int. Cl.: G01D 18/00, G01N 33/00, G08B 29/20, G08B 21/12

(54) **METHOD AND APPARATUS FOR SENSING AND FOR IMPROVING SENSOR ACCURACY**
VERFAHREN UND VORRICHTUNG ZUM MESSEN UND VERBESSERN DER SENSORGENAUIGKEIT
MÉTHODE ET APPAREIL POUR LA MESURE ET L'AMÉLIORATION DE LA PRÉCISION DU CAPTEUR

(30) Priority: 27.06.2016 GB 201611154
(43) Date of publication of application: 01.05.2019
(73) Proprietor: Sensyne Health Group Limited, Oxford OX4 4GE (GB)
(72) Inventor: RANGEL, Manuel Pinuela, London W10 5AD (GB); STEFAN, Diana, London W10 5AD (GB); WILSON, Padarn, London W10 5AD (GB)
(74) Representative: Leach, Sean Adam
(86) International application number: PCT/GB2017/051871
(87) International publication number: WO 2018/002602

(56) References cited:
- US-A1- 2011 316 699
- US-A1- 2014 200 840
- US-A1- 2014 278 186

## Description

The present invention relates to sensing technology, and more particularly to methods and apparatus for improving accuracy of sensor readings.

Recent scientific research indicates that habitual exposure to certain environmental contaminants may have significant adverse effects on health. The incidence of such effects may depend sensitively on the level (for example concentration) of the component. Even below levels which are immediately toxic, some components may damage mental and physical health.

Environmental parameters such as temperature, and pressure, and the level of components in the atmosphere (such as humidity, CO₂, excess O₂, O₃ and other contaminants) may be measured by a variety of techniques. Typically the technique which is chosen depends on the level of accuracy required.

As just one example, domestic carbon monoxide alarms are typically able to detect levels of carbon monoxide of a few hundred parts per million (ppm) and have an accuracy of about ±30 ppm. This level of accuracy may be acceptable when detecting immediately toxic levels of such a contaminant, but is of less use when dealing with levels which may be relevant in attempting to avoid and/or protect against harm caused by habitual exposure to much lower levels. It has been shown that long-term exposure to low levels of carbon monoxide can lead to neurological symptoms, such as difficulty thinking or concentrating and frequent emotional changes - for example, becoming easily irritated, depressed or making impulsive or irrational decisions.

The levels which have been found to give rise to such effects after long periods of habitual exposure may be far below the limit of detectability of many standard detectors. Indeed, the difference between safe and harmful levels may be less than the resolution limits of common detectors.

Providing sensors of increased sensitivity in every area which may be occupied or visited regularly would appear to be the natural answer. However this would require the provision of large numbers of highly accurate sensors. The cost of the units themselves, let alone their installation and maintenance, therefore is prohibitive. Carbon monoxide monitoring is just one example of measurement of an environmental parameter.

A calibration, e.g. a relation between the input and the output of a measuring system may be established by a calibration procedure. This procedure may involve applying a known value, or a series of known values, to a sensor for the purpose of observing its output. By the application of a range of such known values and observation of the sensor output, a calibration can be determined. Such a calibration procedure may improve accuracy of a sensor reading, but is generally time consuming and costly. Therefore, mass produced sensors are typically calibrated at point of production in a uniform fashion - all sensors of a particular type are assumed to have the same sensing characteristic. The alternative, individually calibrating each sensor, would add significant cost and inconvenience. In practice, it may therefore be preferable to simply buy a sensor which provides more accurate data.

US 2014/0278186 A1 discloses a calibration method for a distributed sensor system.

US 2011/0316699 A1 discloses an environmental monitoring device capable of auto or self calibration.

US 2014/0200840 A1 discloses a platform for portable sensing applications.

Aspects and examples of the invention are set out in the appended claims and aim to address at least a part of the above described technical problem.

Embodiments of the present disclosure will now be described, by way of example only, with reference to the accompanying drawings in which:
Figure 1 shows a network comprising a server and a plurality of sensor systems;
Figure 2 is a flow chart which illustrates a method of controlling a sensor system such as one of those shown in Figure 1;
Figure 3 is a flow chart illustrating an example of controlling a network of carbon monoxide sensors according to a method such as that of Figure 2
Figure 4 shows an apparatus comprising a plurality of sensors distributed about a geographic area; and
Figure 5 shows a sensor system comprising a mobile telecommunications handset and a sensor.

In the drawings like reference numerals are used to indicate like elements.

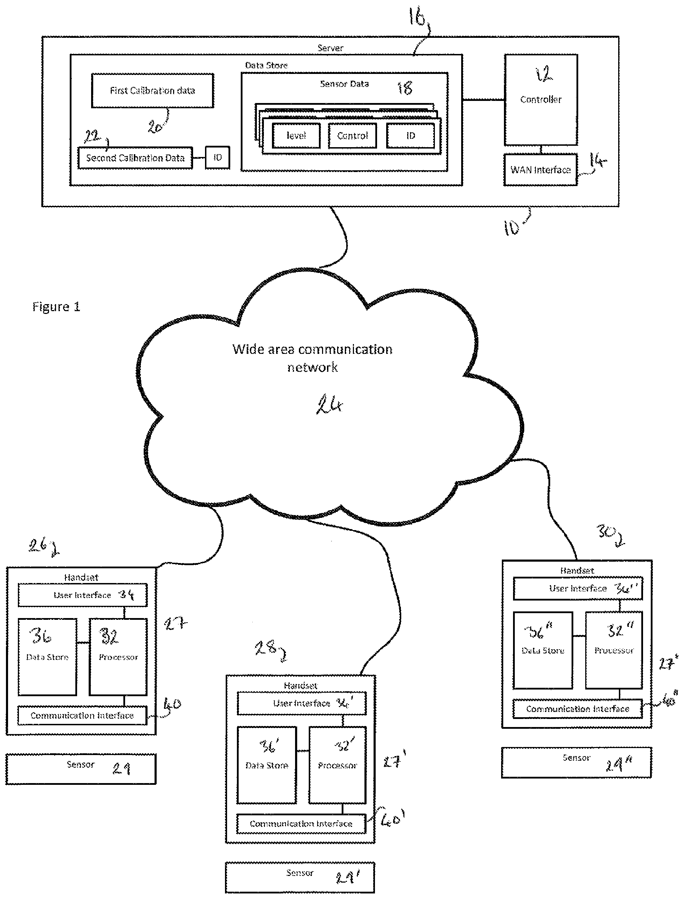

Figure 1 shows a network comprising a server and a plurality of sensor systems 26, 28, 30. The server 10 is coupled to communicate, via a wide area communications network 24 with the sensor systems 26, 28, 30.

When a particular sensor system 26 is first deployed, the server 10 illustrated in Figure 1 uses calibration data derived from the population of other sensor systems 28, 30 to calibrate the sensor data received from that particular sensor. The server 10 then observes the behaviour of that particular sensor system 26 over time to develop a specific calibration for that particular sensor system. The calibration applied by the server 10 may undergo a transition from its initial state (dictated solely by the behaviour of the population) so that over time it gradually shifts to the specific calibration for that particular sensor system. This gradual shift may involve using a weighted combination of the calibration from the population and that from the particular sensor. The weighting may be based on one or more metrics of the reliability of the calibration from the particular sensor.

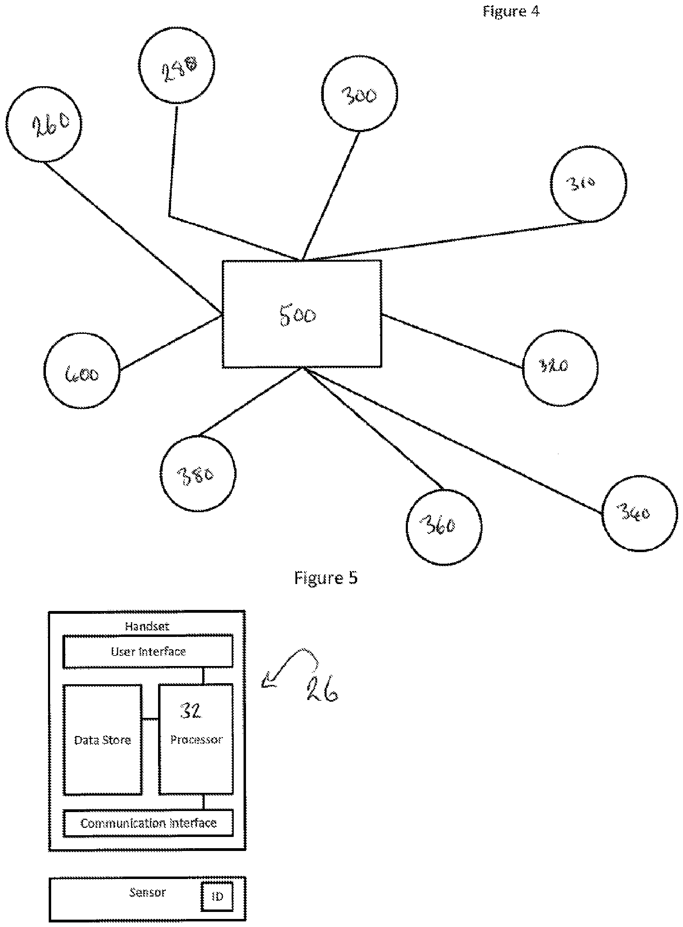

The sensor systems 26, 28, 30 illustrated in Figure 1 each comprise a mobile communications apparatus 27 and a sensor 29. The mobile telecommunications apparatus 27 comprises a processor 32, a data store 36, a communications interface 40 and a user interface. An example of such a sensor system is described in more detail below with reference to Figure 5.

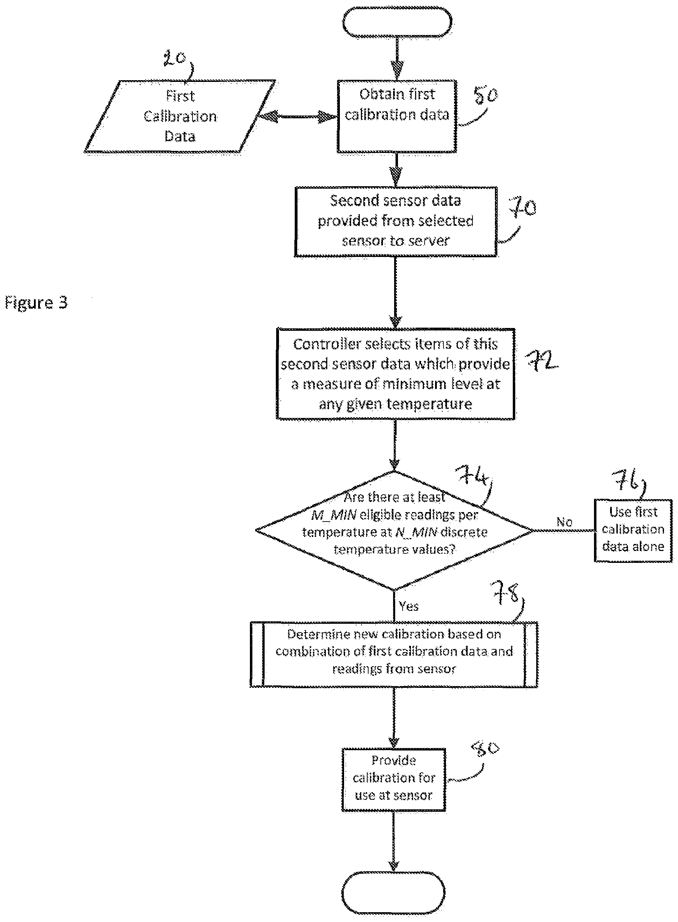

The sensor 29 is arranged to sense an environmental parameter, and to provide the sensed level of that parameter to the mobile communications apparatus 27. The sensor illustrated in Figure 1 is also configured to sense one or more control variables, such as temperature and also to provide this to the mobile communications apparatus 27. Accordingly it will be understood that in addition to the environmental parameter that is to be sensed the data obtained from the sensor may comprise one or more control variables sensed at the time the parameter was measured by the sensor. In the example described below with reference to Figure 3, the control variable is temperature but other control variables may be used.

The mobile communications apparatus 27 is coupled to communicate with the sensor 29 to obtain data from the sensor 29 and to communicate that sensor data over the network to the server 10.

The server 10 shown in Figure 1 comprises a data store 16, a wide area communication interface for communicating over the wide area communications network, and a controller 12. The data store 16 is coupled to the controller 12 to enable the controller 12 to retrieve data from it, and to store new data in it. The controller 12 is coupled to the wide area communications interface 14 for receiving sensor data from sensor system 26, 28, 30 over the network, and for sending data and command signals to those systems.

The wide area communications interface 14 comprises a wired or wireless interface for communication over a packet switched network, such as the internet.

The data store 16 is operable to store data associated with one or more of the sensor systems 26, 28, 30. The information stored generally includes:
i. first calibration data 20 , describing a default calibration for the sensor systems 26, 28, 30;
ii. second calibration data 22 which relates to the calibration for a specific one 26 of the sensor systems 26, 28, 30 and which is related to an identifier of that specific sensor system; and
iii. items of data 18 collected from the sensor systems 26, 28, 30, typically this data includes a level value indicating the level of the sensed parameter, one or more control variables indicating a condition at the sensor when the level was sensed, and an identifier of the sensor system which collected that data.

The controller 12 is configured to obtain items of this sensor data 18 from each of the sensor systems 26, 28, 30 and to record them in the data store 16.

It is also configured to determine a calibration for each sensor system 26 based on the sensor data collected from that sensor system 26 and the first calibration data 20 stored in the data store, and then to use these to select a calibration for that particular sensor.

The controller 12 is configured so that a selected condition may be used to determine whether to use the default calibration, or instead to switch to a more specific calibration for a selected sensor. One example of such a condition is described below with reference to Figure 3, but others may be used. In a simplest case this may comprise checking whether a sufficient number of items of sensor data have been collected from that sensor. It will be appreciated however that further or different conditions may also be applied. As just one example the condition may be whether a sufficient number of levels have been sensed at each of a range of values of a control variable. Where the control variable is sampled at a series of discrete values, then this condition may be expressed as a minimum number of level measurements per value of the control variable.

The controller 12 is also configured so that in the event that the condition for switching away from the default calibration is met, it determines the specific calibration which is to be used as a weighted combination of:
(i) the first calibration data 20 , and
(ii) second calibration data determined based on items of sensor data collected from the sensor in question.

The controller 12 is configured to determine this second calibration data by selecting items of the sensor data, and then fitting a data model for the sensor type to those selected items of sensor data. The selected items of sensor data may comprise an estimate of the minimum sensor level observed at each value of the control variable. In addition, or as an alternative, the items of sensor data may be weighted in this fitting based on when they were obtained. For example, more recently obtained sensor data may be given a greater weighting than older data. For example, data sensed outside a selected time interval may be given a small or zero weighting - this may be achieved by only using sensor data that was selected during a recent interval of some selected length. The length of this interval may be chosen to tune the calibrations sensitivity to sensor drift.

The data model used by the controller to determine this second calibration data relates the control variable values to the sensor data values. Accordingly, the controller can use the model to determine a correction for systematic bias error (for example a zero error or offset) which may vary as a function of the control variable. The data model may also include further components such as variations in one or more other control variables, for example this may account for other specific environmental variables which have a bearing on the sensed data.

It will be appreciated in the context of the present disclosure that this may be applied in a variety of different ways according to the nature of the underlying data and the type of sensor. For example, if a type of sensor is known to exhibit a systematic offset which varies as a function of a particular control variable, then the second calibration data may be obtained by fitting a data model which reflects that function to the items of sensor data. For example, if the variation is known to be a linear one a first order polynomial model can be used, if exponential an exponential model, if a power series then a power series model, and so on. One example of this process is described below with reference to Figure 3, but other such examples are envisaged. However it is achieved, the result of this approach is that the second calibration data comprises a relation between the level sensed by the sensor, and the value of a control variable. Whether this relation is numerical (e.g. in the form of a look-up table, LUT, or other association of data values) or analytic (e.g. in the form of an equation) this relation can be used to determine the influence of the control variable on the level sensed by the sensor. Accordingly, given the value of the control variable this relation can be used to reduce the influence of that control variable on the sensed level. For example it may permit the influence of the control variable to be subtracted away, or otherwise modelled out of the sensed level. Rather than simply using the second calibration data as the calibration for the particular sensor, a weighted combination of the second calibration data (specific to that particular sensor) and the first calibration data (calculated from a population of similar sensors) can be used.

The controller 12 is also configured to determine the weighting based on the data gathered from the particular sensor. This may be done by applying a weighting which is based on the number of samples of sensor data used to calculate the second calibration data (e.g. a simple count of the items of data used in the fit) . More sophisticated approaches may also be used. For example, the count of data items may be restricted to those items which match a quality criterion.

This quality criterion may be a maximum deviation from an expected level. For example - the expected level may be based on the model fitted to the data items to determine the second calibration data, and outliers which deviate from that fit by more than some maximum threshold may not be taken into account when calculating the count of data items.

In addition to, or as an alternative to, using a weighting based on the count of the data items, the weighting may be based on known or expected characteristics of the calibration data for a particular sensor. For example - it may be known that the particular sensor has a monotonic calibration function, so the controller 12 may be configured to reduce the weighting applied to the second calibration data in the event that it deviates from monotonicity. As another example, the controller 12 may be configured to select the weighting based on the value of the second calibration data, e.g. based on its value in a particular range of the control variable. In addition, or as an alternative, a similar approach may be applied using the gradient of the second calibration data. One such approach will be described below with reference to Figure 3 which describes using the low temperature behaviour of a fitted calibration to determine the weighting to be applied to that fitted calibration when combining it with a default, population based, estimate.

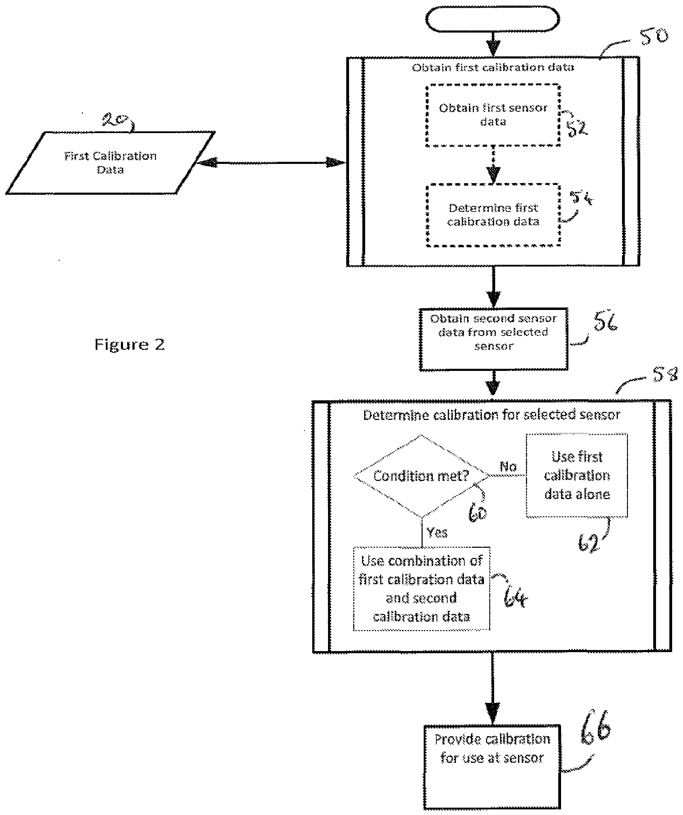

Operation of the apparatus shown in Figure 1 will now be described with reference to Figure 2.

As illustrated in Figure 2, to obtain 50 first calibration data the server 10 controller 12 can communicate with each of the plurality of sensor systems 26, 28, 30 to obtain 52 sensor data from each of them. This first sensor data 20 generally comprises a sensed level, and an indication of a control variable at the sensor which provided it when the data was collected. The controller 12 can then determine 54 first calibration data based on this first sensor data - for example by fitting a function of the control variable to the first sensor data.

The controller 12 can then store the first calibration data into the data store. Of course, if it has already been determined the first calibration data can simply be obtained 50 by retrieving it from the data store. Likewise, first calibration data stored in the data store 16 may be updated based on observations obtained from sensor systems 26, 28, 30. This may be done at intervals, e.g. periodically and/or in response to some trigger condition. The trigger condition may be an increase in the number of sensor systems of more than some threshold level - for example at least 5%, for example at least 10%.

Then, a plurality of items of second sensor data is obtained 56 over the wide area communications network 24 from a selected sensor system 26. Each such item of second sensor data indicates the level of an environmental parameter sensed by the selected sensor and a value of a control variable at the time that level was sensed.

Using this second sensor data and the first calibration data 20, the controller 12 of the server 10 can determine 58 a calibration for use in correcting sensor data collected by the selected sensor system. This may be done according to the approach described above with reference to Figure 1 whereby if a basic first condition 60 is not met, the first calibration data 20 is used to set a default calibration for use in correcting the sensor data. In the alternative, if that condition is met, a combination of the first calibration data and some second calibration data may be used 64. That combination may be weighted according to any one or more of the approaches described above.

The calibration can be provided 66, over the network, to the sensor system 26 - for example by being sent in a network message. The processor 32 of the sensor system 26 may store the calibration into the sensor system's data store 36 - for example in the form of a data table or other stored association. To indicate the level of the parameter at the sensor, the processor 32 obtains a level of the sensed parameter from the sensor and obtains an associated value of the control variable. The processor 32 then uses the control variable value and the calibration to correct the sensed level value. This corrected level data can be provided to the user interface 34 at the sensor system 26 and/or it may be used to provide an indication of the level of the parameter at the location of that system. The server 10 may communicate with the set of sensor systems 26, 28, 30 and use the sensed level values at the location of each of the sensor systems 26, 28, 30 to assemble a map of the parameter level across a geographic area. In some embodiments the sensor system 26 may send the sensor data to the server, and the controller at the server can then use the control variable value associated with that sensor data and the calibration to correct the sensed level value at the server. The corrected level value can then be transmitted back to the sensor system.

Figure 3 illustrates an example of this method applied to a carbon monoxide sensor which exhibits a temperature dependent zero offset - referred to herein as a baseline.

In the examples described with reference to Figure 3, the carbon monoxide sensor comprises a screen printed electrochemical sensor having: a measurement range of 0 to 1,000 ppm; a detection limit of 0.5 ppm; a resolution of < 100 ppb; an operating temperature range of -30 to 55 °C (-20 to 40 °C continuous recommended) ; and Operating Humidity Range (non-condensing) of 15 to 95% recommended continuous and 0 to >95% RH - intermittent. One such sensor is available from Spec Sensors LLC, 8430 Central Ave., Suite D Newark, CA 94560 under product number 3SP_CO_1000 Package 110-109. But this same method may be applied to any other sensor having a temperature dependent baseline offset (zero error). It will also be appreciated in the context of the present disclosure that this is merely one example of implementation of a system such as that described above with reference to Figure 1 and Figure 2.

As with those examples, when a particular sensor is deployed the calibration used for the sensor is based on first calibration data. This is, in effect a default calibration, which may have been calculated from sensor data obtained from a population of similar sensor systems 26, 28, 30. Over time, as data is obtained from that particular sensor, it transitions to a state in which the calibration is based on a weighted combination of the first calibration data and second calibration data learned from that particular sensor.

After a sensor is deployed, the method illustrated in Figure 3 proceeds as follows. The first calibration data 20 is obtained 50, either from a data store or by calculating it from first sensor data indicative of a level of an environmental contaminant at each of a plurality of sensors. This first calibration data comprises a relation between temperature and the signal level provided by the sensors, at a range of temperatures. Irrespective of how it is obtained, this first calibration data may represent a zero level offset as a function of temperature - an expected level of the sensor output in the presence of a zero carbon monoxide level at each a range of different temperature values.

As it continues to operate, the sensor system 26 provides 70 items of sensor data to the server 10. These items of sensor data comprise a carbon monoxide level and a corresponding temperature indicating the temperature at which the CO level was sensed. The controller 12 of the server determines 72 a measure of the minimum level of carbon monoxide at any given temperature. This may be done by selecting a group of the lowest values at each temperature and determining the minimum level based on those selected values - for example it may be based on an average of those selected values such as the mean or median or some weighted combination of those selected values.

This provides an estimate of the zero level of the sensor at any given temperature - a floor level estimate. The items of data on which it is based, and the associated floor level estimate may be stored in the data store 16 at the server.

The controller 12 then determines 74 whether a sufficient number of items of sensor data (referred to as "second sensor data") have been collected from the particular sensor system 26 for this estimate of the minimum level to be treated as reliable. It does this by determining whether a threshold number of carbon monoxide level readings have been collected at each of a set of selected temperature values. Based on this first condition, the controller 12 determines whether to switch from the use of the default calibration alone toward a calibration based on the second sensor data. If at least a minimum count of items of second sensor data have been collected per value of the control variable (temperature in this case), it can switch and if not it may continue to use the default calibration until a sufficient number density of data points has been collected.

The items of second sensor data which count towards meeting this first condition are limited to those which meet an eligibility criterion. Items of sensor data which do not lie within a selected range of values may be rejected as ineligible. This range may be selected based on the physical characteristics of the device - for example non-physical or out of range values maybe disregarded. The selected range of values may be fixed, and/or it may be chosen based on fitting a data model to the second sensor data. For example, the controller 12 may also determine an eligibility criterion by fitting a data model (such as an exponential curve) to the second sensor data as a function of temperature and selecting a threshold deviation about this data model. Items of data which do not fall within this range are excluded from the second sensor data. These two eligibility criteria may be applied in combination - for example values outside a fixed range may be discarded before a data model is fitted to the remaining values, and then the outliers which exceed a threshold deviation from that fitted model can also be deemed ineligible.

In the event that the remaining (eligible) samples do not provide, for each of the set of discrete temperature values, the threshold number of carbon monoxide level readings the first condition is deemed not met and the sensor system 26 continues 76 to operate using a calibration based on the first calibration data alone.

In the alternative, in the event that the eligible items of second sensor data meet the first condition, the controller 12 determines 78 a new calibration for the sensor system 26 based on both the first calibration data and the eligible items of sensor data collected from that sensor system.

To calculate this combination the controller 12 fits an exponential curve to the first calibration data and the floor values of the second sensor data at each temperature. To determine this fit, the controller 12 weights the contribution of the second sensor data according to one or more of the following:
- A raw count of the items of second sensor data, for example a weighting at each temperature value based on the number of items of second sensor data collected at that temperature. This can enable reliable measures of the floor level (determined based on many repeated observations) to be given greater weight than those determined based on a smaller number of observations.
- The count of the items of second sensor data which fulfil a first quality criterion, such as that described above with reference to eligibility. For example - the quality criterion may be a maximum deviation from an expected level of the contaminant. This can enable the weighting to be reduced if the data includes spikes or other anomalies.
- The gradient and/or the value, in a selected range of temperatures, of a curve fitted to the floor values of the second sensor data. This can enable the weighting to be reduced if the characteristics of the sensor determined for observation do not match known characteristics of that sensor type. For example, if the gradient in a low temperature region, between say 0°C and 5°C exceeds a certain threshold and/or is negative, the weighting may be reduced or set to zero. If on the other hand the curve which fits the data behaves as expected the weighting can be increased.
- Monotonicity of the curve fitted to the floor values of the second sensor data. If the curve appears non-monotonic, for example if it does not always increase in value with increasing temperature, the controller 12 may reduce the weighting. The monotonicity may be assessed based on the sign of coefficients obtained from fitting the exponential model to obtain the curve. This can avoid the need to examine the entire data series for monotonicity.

Once the weighting has been determined, the controller 12 determines the fit using the weighting to determine a calibration baseline which takes account of both the known characteristics of a population of similar sensors and the particular characteristics of the sensor itself. This calibration baseline can then be employed at the server 10 to correct sensor data obtained from that sensor. In addition, or as an alternative, the calibration can be provided 80 to the sensor system. The sensor system 26 can then use the calibration and a measure of the temperature to determine a correction for the sensed level.

If the sensor system 26 also provides location information, indicating its geographic location, to the server 10 the server 10 can assemble a map based on the sensor level values and this geographic information to provide a geographic map of the sensed level - e.g. by interpolating between locations at which sensors are deployed. The map may be updated in response to receiving new sensor data from the sensors.

Figure 4 shows an apparatus comprising a plurality of sensing devices 260, 280, 300, 310, 320, 340, 360, 380, 400 distributed about a geographic area, and a controller 500 in communication with those sensors.

The sensing devices 260 - 400 are configured to sense the level of an environmental parameter, such as a contaminant level. The sensing devices 260 - 400 are also configured to provide data indicating a value of at least one control variable associated with the sensed level values. The sensing devices are arranged to provide this data to the controller 500.

The controller 500 is coupled to communicate with each of the sensing devices 260 - 400 for obtaining sensor data. The controller is configured to store calibration data for each of the sensing devices - generally this calibration data comprises a relation between a control variable and a sensor signal level.

The controller 500 also stores an association between an identifier of each sensing device and location information indicating a location of that sensing device. In some cases, this information may be provided from the sensing devices to the controller 500 when they are deployed or at other intervals.

In operation, the controller 500 obtains first sensor data from each of the sensing devices, and uses this first sensor data to determine a calibration which accounts for a variation in at least one of the control variables. This may be done by fitting a selected data model to the sensor data using the associated control variable data. A further sensing device 400 is then deployed into the geographic area across which the other sensing devices are distributed. The controller obtains sensor data and associated control variable values from this further sensing device 400. This provides data indicative of the level of the environmental parameter at the geographic location of the selected sensor - this location of course may be different from the specific locations of the other individual sensing device.

The controller 500 then determines, based on the calibration data, the control variable values and the data from the further sensing device, the level of the environmental parameter at the geographic location of the further sensing device.

The calibration data generally comprises a combination of (a) the first set of calibration data, determined based on data collected from the group of sensing devices already deployed in the geographic area; and (b) a further (or second) calibration data determined based on sensor data obtained just from the further sensing device that has been deployed into the area. The two types of calibration data can be combined using a weighting of the second calibration data which increases with a count of the items of the second sensor data. This count may be restricted to items which meet some eligibility criterion as explained elsewhere herein.

In addition, this architecture may be used to implement the methods described above with reference to Figure 2 and/or Figure 3 without the need for mobile communications devices or the use of a separate wide area communications network. As with those examples, it will be appreciated that such apparatus may be used to monitor carbon monoxide levels and to control for variations in temperature which may affect the sensors used in those approaches.

It will be appreciated in the context of the present disclosure that the sensing devices described with reference to Figure 4 may comprise sensor systems such as those described with reference to Figure 1 and Figure 5. One example of such a system is a sensor with a short range communication link, e.g. a wireless link such as Bluetooth (RTM) to a communications apparatus configured to communicate over a wide area network with the controller 500. Examples of such communications apparatus include a Bluetooth (RTM) gateway. The sensor device may instead just comprise the sensors with a basic communication interface arranged to provide sensor signals direct to the controller 500.

Figure 5 shows a sensor system 26 comprising a mobile telecommunications handset 27 and a sensor 29. Such sensor systems may be used in the apparatus and methods described with reference to Figure 1 and Figure 2.

The mobile telecommunications handset 27 comprises a processor 32, a data store 36, a communications interface 40 and a user interface.

The processor 32 is coupled to the data store 36 for reading and writing data, and is arranged for communicating via the communications interface 40. The user interface 34 comprises a display controlled by the processor 32 and an input device for receiving operator commands.

The communications interface comprises at least one local channel, such as a short range radio (for example Bluetooth RTM) for communicating with the sensor and a wide area channel for communicating over a wide area communications network 24 such as a cellular telephone network and/or the internet.

The sensor is configured to sense a level of an environmental parameter and a control variable and comprises machine readable identifier data. The sensor is operable to communicate via the local channel for providing the sensed level and control variable data to the handset. The handset is also able to obtain the identifier data from the sensor. This may also be done by the local channel or by the handset otherwise reading the identifier data from the sensor - for example it may be read using a barcode reader or QR code reader, or using optical character recognition software.

In operation, the handset obtains this identifier from the sensor, and operates the communication interface to transmit the unique identifier over a wide area communications network 24 from the handset to a remote device - for example to a server 10 such as that illustrated in Figure 1. The handset then receives over the wide area communications network 24 from this remote device, a calibration for the sensor.

In the first instance this calibration is based solely on a set of first calibration data. This first calibration data having been determined based on first sensor data obtained from each of a plurality of similar sensors configured to sense the level of the environmental parameter. It will be appreciated in the context of the present disclosure that the remote device may have selected this first calibration data based on the unique identifier - for example by identifying the type of sensor and then selecting from amongst one of a set of stored calibrations.

The sensor at the handset then senses a plurality of items of second sensor data indicating the level of the environmental parameter, and optionally also the level of a control variable. The processor 32 of the handset then provides an indication of the level of the parameter at the handset based on the calibration and this second sensor data.

The handset may also transmit the plurality of items of second sensor data to the remote device. In response the remote device may determine a revised calibration based on this second sensor data. This revised calibration can then be used to update the calibration used at the handset for indicating the level of the parameter. This revised, updated, calibration takes account of the first calibration data and also the second sensor data - this may be done based on the second sensor data itself, or using a calibration curve fitted to that data which can then be combined with the first calibration data. This may be done using a weighting such as any one or more of the weightings described herein.

Although Figure 3, and some of the other drawings have been described with reference to a carbon monoxide sensor the same approach may be used in the adjustment of data from any appropriate sensor. The sensors may comprise a group of similar sensors each arranged to sense an environmental parameter, such as humidity, or another type of parameter such as the level of a contaminant such as an airborne particulate such as pollen or dust, an aerosol such as pesticide, or a gaseous contaminant such as NOₓ.

The first calibration data may be calculated as part of the methods described herein, for example by sampling sensor data from a population of similar sensor systems and estimating the baseline or other systematic correction for a particular control variable based on the sensor data collected from that population. Accordingly, in some embodiments of the disclosure the first calibration data is updated at intervals based on measurements collected from the population of sensors. In some embodiments however the first calibration data may be fixed, for example it may comprise a stored data value which need not be updated.

Where a calibration is determined based on the combination of the first calibration data and data learned from a particular sensor it may be determined by weighting the combination of the sensor data itself with the first calibration data, or by first fitting a data model to the sensor data to obtain second calibration data (the fit of the model to that sensor data) and then determining a weighted combination of this fit with the first calibration data.

The example described with reference to Figure 3 uses temperature as a control variable, but additional or alternative control variables may also be taken account of in this way. Examples of control variables which may be calibrated for include temperature, humidity, pressure, light levels, acceleration, speed, local gravity, or any other control variable which can be sensed concurrently with the level that is to be sensed. Examples of such other control variables include orientation, location, altitude, and the level of other contaminants such as volatile hydrocarbons.

The control variable values may be provided by a component of the sensor, or by any other device arranged to determine their value. For example, a separate or integrated device may be provided at or in the vicinity of the sensor system. The control variable values and/or the sensor data may be provided with an identifier of the sensor with which they are associated.

The calibration described with reference to Figure 3 is a baseline or zero level correction, but other types of systematic error and bias may be addressed in this way.

In the example described with reference to Figure 3, the items of second sensor data which count towards meeting the condition for switching away from the default baseline correction are limited to those which meet a selected eligibility criterion. In some examples however all the samples obtained from the sensor may be used. In the example of Figure 3 the eligibility criterion is based on a threshold deviation about a data model fitted to the second sensor data. More simple eligibility criteria may be applied. For example, the eligibility criterion may exclude values which deviate from an average (such as the median or mean) by more than a selected maximum limit. This may be the mean or median associated with a particular value of the control variable (e.g. a particular temperature), or a particular range of the control variable, or it may be the global average across all values of the control variable. The maximum limit may be selected based on a measure of the spread of the data values such as the variance.

The calibration described with reference to Figure 3 is provided by fitting an exponential function of the control variable to the floor values of the second sensor data to provide a baseline correction. However, other data models may be used. In some embodiments the data model is selected based on the type of the sensor. For example, the sensor may be identified by data obtained over the network and the data model used to identify the calibration is selected to match. The server 10 may store an association between each of a plurality of types of sensor and one or more corresponding data models. This can enable the sensor to select an appropriate data model based on information identifying the sensor.

The fitting procedures described herein may comprise linear regression, or may be based on non-linear merit functions. In some embodiments log-linear fitting is used. The nature of the fitting procedure and/or the merit function employed in any particular instance is selected based on the data model which is to be fitted to the sensor data. Examples of suitable fitting procedures may be found in Numerical Recipes in C; The Art of Scientific Computing; Second Edition Published 2002 by the Press Syndicate of the University of Cambridge, The Pitt Building, Trumpington Street, Cambridge CB2 1RP.

The wide area network described herein may be provided by any geographically dispersed telecommunications network. It may be packet switched and may comprise, at least in part, a cellular telecommunications network. The wide area communications interfaces described herein comprise any interface operable to communicate over such a network. Examples of such interfaces comprise modems for communication over packet switched networks, which may comprise wired and/or wireless components. Such interfaces may also comprise GSM, GPRS, 3GPP, LTE and other mobile communications interfaces.

The mobile telecommunications device illustrated in Figure 1 and Figure 5 has been described as a handset, but it will be appreciated in the context of the present disclosure that this encompasses any user equipment (UE) for communicating over a wide area network and having the necessary data processing capability. It can be a hand-held telephone, a laptop computer equipped with a mobile broadband adapter, a tablet computer, a Bluetooth gateway, a specifically designed electronic communications apparatus, or any other device. It will be appreciated that such devices may be configured to determine their own location, for example using global positioning systems GPS devices and/or based on other methods such as using information from WLAN signals and telecommunications signals.

With reference to the drawings in general, it will be appreciated that schematic functional block diagrams are used to indicate functionality of systems and apparatus described herein. It will be appreciated however that the functionality need not be divided in this way, and should not be taken to imply any particular structure of hardware other than that described and claimed below. The function of one or more of the elements shown in the drawings may be further subdivided, and/or distributed throughout apparatus of the disclosure. In some embodiments the function of one or more elements shown in the drawings may be integrated into a single functional unit. For example the functionality of the sensor and mobile telecommunications apparatus in the sensor system may be integrated into a single apparatus, or differently subdivided between two or more separable devices.

The above embodiments are to be understood as illustrative examples. Further embodiments are envisaged. For example, in an embodiment the disclosure provides a method of controlling a sensor system, the method comprising: obtaining, from each of a plurality of sensors, first sensor data indicative of a level of an environmental contaminant at each of the plurality of sensors; determining first calibration data based on the first sensor data; obtaining, over a wide area communications network from a selected sensor, a plurality of items of second sensor data each indicative of the level of the environmental contaminant sensed by the selected sensor; determining a calibration for the selected sensor based on the second sensor data and the first calibration data; and providing an indication of the level of the contaminant at the selected sensor, wherein the indication is corrected using the calibration. Providing the indication may comprise sending the calibration over the wide area communications network to the sensor system which comprises the selected sensor so that the processor of the sensor system can determine the correction. Alternatively, providing the indication may comprise sending the sensor data over the wide area communications network to the server so the server can apply the calibration to correct the sensor data. This corrected sensor data may, optionally, be sent back to the sensor system. It is to be understood that any feature described in relation to any one embodiment (such as those described in this paragraph) may be used alone, or in combination with other features described elsewhere herein, and may also be used in combination with one or more features of any other of the embodiments, or any combination of any other of the embodiments. Furthermore, equivalents and modifications not described above may also be employed without departing from the scope of the invention, which is defined in the accompanying claims.

In some embodiments the sensor may be provided with an indication of its sensitivity characteristics. This may be carried by the sensor itself, for example in the form of a machine readable marker. Embodiments of the disclosure may comprise obtaining this indication and using it to select the first calibration data from a set of stored sensitivity characteristics. This can enable the first calibration data to provide a better starting point for the calibration for any given sensor.

Having read the present disclosure it will however be appreciated that the method disclosed herein can be implemented without knowing details of the sensor - i.e. there is no need for the server to know that a CO sensor is being used or to know that that sensor has a certain sensitivity.

It will also be appreciated in the context of the present disclosure that where reference is made to values of a control variable this may relate to discrete values, or to ranges of such values such as histogram bins. For example, the first condition used to determine whether a sufficient number of sensor data values have been observed could be based on the number of data values in each of a series of bins - e.g. bins 3-5, 5-7, 7-9 instead of 4,6,8. The bins could overlap i.e. 3-5, 4-6, 5-7 etc. These numeric values and the bin widths are of course merely exemplary - other values and ranges can be used.

In some examples, one or more memory elements can store data and/or program instructions used to implement the operations described herein. Embodiments of the disclosure provide tangible, non-transitory storage media comprising program instructions operable to program a processor to perform any one or more of the methods described and/or claimed herein and/or to provide data processing apparatus as described and/or claimed herein. The data stores described herein may comprise volatile and/or non-volatile memory for storing computer readable data and instructions.

The processors and controllers described herein (and the activities they perform) may be implemented with fixed logic such as assemblies of logic gates or programmable logic such as software and/or computer program instructions executed by a processor. Other kinds of programmable logic include programmable processors, programmable digital logic (e.g., a field programmable gate array (FPGA), an erasable programmable read only memory (EPROM), an electrically erasable programmable read only memory (EEPROM)), an application specific integrated circuit, ASIC, or any other kind of digital logic, software, code, electronic instructions, flash memory, optical disks, CD-ROMs, DVD ROMs, magnetic or optical cards, other types of machine-readable mediums suitable for storing electronic instructions, or any suitable combination thereof.

## Claims

1. A method of sensing an environmental parameter comprising:
selecting first calibration data (20) for a sensor system (26, 28, 30), wherein the sensor system (26, 28, 30) is configured to provide sensor data (18) indicating a sensed level of the environmental parameter;
providing the first calibration data (20) to the sensor system (26, 28, 30) for use in indicating the environmental parameter;
obtaining, from the sensor system (26, 28, 30), a plurality of items of the sensor data (18);
determining whether a first condition is met by the plurality of items of the sensor data (18);
and, in the event that the first condition is met, sending updated calibration data to the sensor system (26, 28, 30) for use in indicating the environmental parameter,
wherein the updated calibration data is based on the first calibration data (20) and the plurality of items of the sensor data (18).

2. The method of claim 1 further comprising determining the updated calibration data based on a weighted combination of:
(i) the first calibration data (20), and
(ii) second calibration data based on the plurality of items of the sensor data (18).

3. The method of claim 2 wherein the weighting is based on a count of the plurality of items of the second sensor data.

4. The method of claim 3 wherein the count comprises a count of the items which fulfil a first quality criterion.

5. The method of claim 4 wherein the first quality criterion comprises a maximum deviation from an expected level of the contaminant.

6. The method of any of claims 2 to 5 wherein the weighting is based on at least one of:
(i) a gradient; and
(ii) a value;
of the second calibration data in a selected range of a control variable.

7. The method of any of claims 2 to 6 wherein the second calibration data comprises a relation between a control parameter and a sensor signal indicative of the level of the environmental parameter.

8. The method of claim 7 comprising fitting a selected data model to the second sensor data to determine the relation.

9. The method of any of claims 1 to 8 wherein selecting the first calibration data (20) further comprises obtaining, from each of a plurality of sensors, first sensor data (18) indicative of a level of the environmental parameter at each of the plurality of sensors;
and selecting calibration data which relates said first sensor data (18) to a control variable.

10. The method of any of claims 1 to 9 comprising
receiving identifier data from the sensor system (26, 28, 30) ;
and selecting the first calibration data (20) based on the identifier data.

11. The method of any preceding claim, wherein the environmental parameter comprises at least one of: (i) temperature, (ii) pressure, (iii) a level of components in the atmosphere, (iv) humidity, (v) the level of a contaminant for example an airborne particulate such as NOₓ, (vi) carbon monoxide.

12. The method of any preceding claim, wherein obtaining a plurality of items of the sensor data (18) from the sensor system (26, 28, 30) further comprises obtaining control variable data relating to the environmental parameter.

13. The method of claim 12, wherein the control variable data comprises at least one of: (i) a location of the sensor system (26, 28, 30), (ii) temperature, (iii) pressure, (iv) light levels, (v) acceleration, (vi) speed, (vii) local gravity, (viii) orientation, (ix) altitude, (x) level of contaminant such as volatile hydrocarbons and (xi) humidity.

14. The method of any preceding claim, wherein obtain the plurality of items of the sensor data (18) from the sensor system (26, 28, 30) comprises a mobile communications apparatus (27) obtaining data from the sensor system (26, 28, 30) over the network.

15. A computer program product comprising program instructions configured to program a processor to perform the method of any of claims 1 to 14.

## Patentansprüche

1. Ein Verfahren zum Sensieren eines Umweltparameters, das Folgendes umfasst:
Auswählen erster Kalibrierungsdaten (20) für ein Sensorsystem (26, 28, 30), wobei das Sensorsystem (26, 28, 30) konfiguriert ist, um Sensordaten (18) bereitzustellen, die einen sensierten Pegel des Umweltparameters anzeigen;
Bereitstellen, dem Sensorsystem (26, 28, 30), der ersten Kalibrierungsdaten (20) zur Verwendung beim Anzeigen des Umweltparameters;
Erhalten, von dem Sensorsystem (26, 28, 30), einer Vielzahl von Elementen der Sensordaten (18);
Bestimmen, ob eine erste Bedingung von der Vielzahl von Elementen der Sensordaten (18) eingehalten wird;
und, in dem Fall dass die erste Bedingung eingehalten wird, Senden aktualisierter Kalibrierungsdaten an das Sensorsystem (26, 28, 30) zur Verwendung beim Anzeigen des Umweltparameters,
wobei die aktualisierten Kalibrierungsdaten auf den ersten Kalibrierungsdaten (20) und der Vielzahl von Elementen der Sensordaten (18) basieren.

2. Verfahren nach Anspruch 1, das ferner das Bestimmen der aktualisierten Kalibrierungsdaten basierend auf einer gewichteten Kombination von Folgendem umfasst:
(i) den ersten Kalibrierungsdaten (20) und
(ii) zweiten Kalibrierungsdaten basierend auf der Vielzahl von Elementen der Sensordaten (18).

3. Verfahren nach Anspruch 2, wobei das Gewichten auf einer Zählung der Vielzahl von Elementen der zweiten Sensordaten basiert.

4. Verfahren nach Anspruch 3, wobei die Zählung eine Zählung der Elemente umfasst, die ein erstes Qualitätskriterium erfüllen.

5. Verfahren nach Anspruch 4, wobei das erste Qualitätskriterium eine maximale Abweichung von einem erwarteten Pegel des Kontaminanten umfasst.

6. Verfahren nach einem der Ansprüche 2 bis 5, wobei das Gewichten auf mindestens einem von Folgendem basiert:
(i) einem Gradienten; und
(ii) einem Wert;
der zweiten Kalibrierungsdaten in einem ausgewählten Bereich einer Steuerungsgröße.

7. Verfahren nach einem der Ansprüche 2 bis 6, wobei die zweiten Kalibrierungsdaten eine Beziehung zwischen einem Steuerungsparameter und einem Sensorsignal umfasst, wodurch der Pegel des Umweltparameters angegeben wird.

8. Verfahren nach Anspruch 7, das das Anpassen eines ausgewählten Datenmodells an die zweiten Sensordaten umfasst, um die Beziehung zu bestimmen.

9. Verfahren nach einem der Ansprüche 1 bis 8, wobei das Auswählen der ersten Kalibrierungsdaten (20) ferner das Erhalten, von jedem einer Vielzahl von Sensoren, erster Sensordaten (18) umfasst, wodurch ein Pegel des Umweltparameters an jedem der Vielzahl von Sensoren angegeben wird;
und Auswählen von Kalibrierungsdaten, die die genannten ersten Sensordaten (18) mit einer Steuerungsgröße in Bezug bringen.

10. Verfahren nach einem der Ansprüche 1 bis 9, das ferner Folgendes umfasst:
Empfangen von Identifikatordaten von dem Sensorsystem (26, 28, 30),
und Auswählen der ersten Kalibrierungsdaten (20) basierend auf den Identifikatordaten.

11. Verfahren nach einem vorhergehenden Anspruch, wobei der Umweltparameter mindestens eines von Folgendem umfasst: (i) Temperatur, (ii) Druck, (iii) einen Pegel von Komponenten in der Atmosphäre, (iv) Feuchtigkeit, (v) den Pegel eines Kontaminanten, zum Beispiel eines in der Luft schwebenden Partikels, wie etwa NOₓ, (vi) Kohlenmonoxid.

12. Verfahren nach einem vorhergehenden Anspruch, wobei das Erhalten einer Vielzahl von Elementen der Sensordaten (18) von dem Sensorsystem (26, 28, 30) ferner das Erhalten von Steuerungsgrößendaten umfasst, die sich auf den Umweltparameter beziehen.

13. Verfahren nach Anspruch 12, wobei die Daten mindestens eines von Folgendem umfassen: (i) einen Ort des Sensorsystems (26, 28, 30), (ii) Temperatur, (iii) Druck, (iv) Lichtpegel, (v) Beschleunigung, (vi) Geschwindigkeit, (vii) lokale Gravitation, (viii) Orientierung, (ix) Höhe, (x) Pegel von Kontaminant, wie etwa flüchtige Kohlenwasserstoffe und (xi) Feuchtigkeit.

14. Verfahren nach einem vorhergehenden Anspruch, wobei das Erhalten der Vielzahl von Elementen der Sensordaten (18) von dem Sensorsystem (26, 28, 30) eine mobile Kommunikationsvorrichtung (27) umfasst, die Daten über das Netzwerk von dem Sensorsystem (26, 28, 30) erhält.

15. Computerprogrammprodukt, das Programmanweisungen umfasst, die konfiguriert sind, um einen Prozessor zu programmieren, um das Verfahren nach einem der Ansprüche 1 bis 14 durchzuführen.

## Revendications

1. Procédé de détection d'un paramètre environnemental comprenant de :
sélectionner de premières données d'étalonnage (20) pour un système de capteur (26, 28, 30), dans lequel le système de capteur (26, 28, 30) est configuré pour fournir des données de capteur (18) indiquant un niveau détecté du paramètre environnemental;
fournir les premières données d'étalonnage (20) au système de capteur (26, 28, 30) pour une utilisation dans l'indication du paramètre environnemental;
obtenir, à partir du système de capteur (26, 28, 30), une pluralité d'éléments des données de capteur (18);
déterminer si une première condition est satisfaite par la pluralité d'éléments des données de capteur (18) ;
et, dans le cas où la première condition est satisfaite, envoyer des données d'étalonnage mises à jour au système de capteur (26, 28, 30) pour une utilisation dans l'indication du paramètre environnemental,
dans lequel les données d'étalonnage mises à jour sont basées sur les premières données d'étalonnage (20) et sur la pluralité d'éléments des données de capteur (18).

2. Procédé selon la revendication 1, comprenant en outre la détermination des données d'étalonnage mises à jour sur la base d'une combinaison pondérée de :
(i) les premières données d'étalonnage (20), et
(ii) de secondes données d'étalonnage basées sur la pluralité d'éléments des données de capteur (18).

3. Procédé selon la revendication 2, dans lequel la pondération est basée sur un comptage de la pluralité d'éléments des secondes données de capteur.

4. Procédé selon la revendication 3, dans lequel le comptage comprend un comptage des éléments qui remplissent un premier critère de qualité.

5. Procédé selon la revendication 4, dans lequel le premier critère de qualité comprend un écart maximal par rapport à un niveau attendu du contaminant.

6. Procédé selon l'une quelconque des revendications 2 à 5, dans lequel la pondération est basée sur au moins l'un de :
(i) un gradient; et
(ii) une valeur;
des secondes données d'étalonnage dans une plage sélectionnée d'une variable de commande.

7. Procédé selon l'une quelconque des revendications 2 à 6, dans lequel les secondes données d'étalonnage comprennent une relation entre un paramètre de commande et un signal de capteur indiquant le niveau du paramètre environnemental.

8. Procédé selon la revendication 7, comprenant l'adaptation d'un modèle de données sélectionné aux secondes données de capteur pour déterminer la relation.

9. Procédé selon l'une quelconque des revendications 1 à 8, dans lequel la sélection des premières données d'étalonnage (20) comprend en outre l'obtention, à partir de chacun d'une pluralité de capteurs, de premières données de capteur (18) indicatives d'un niveau du paramètre environnemental au niveau de chacun de la pluralité de capteurs;
et sélectionner des données d'étalonnage qui relient lesdites premières données de capteur (18) à une variable de commande.

10. Procédé selon l'une quelconque des revendications 1 à 9, comprenant de :
recevoir des données d'identificateur à partir du système de capteur (26, 28, 30) ;
et sélectionner les premières données d'étalonnage (20) sur la base des données d'identificateur.

11. Procédé selon l'une quelconque des revendications précédentes, dans lequel le paramètre environnemental comprend au moins l'un parmi : (i) la température, (ii) la pression, (iii) un niveau de composants dans l'atmosphère, (iv) l'humidité, (v) le niveau d'un contaminant, par exemple une particule en suspension dans l'air comme le NOₓ, (vi) le monoxyde de carbone.

12. Procédé selon l'une quelconque des revendications précédentes, dans lequel l'obtention d'une pluralité d'éléments des données de capteur (18) à partir du système de capteur (26, 28, 30) comprend en outre l'obtention de données de variable de commande relatives au paramètre environnemental.

13. Procédé selon la revendication 12, dans lequel les données de variable de commande comprennent au moins l'un parmi : (i) une position du système de capteur (26, 28, 30), (ii) la température, (iii) la pression, (iv) les niveaux de lumière, (v) l'accélération, (vi) la vitesse, (vii) la gravité locale, (viii) l'orientation, (ix) l'altitude, (x) le niveau de contaminant tel que les hydrocarbures volatils et (xi) l'humidité.

14. Procédé selon l'une quelconque des revendications précédentes, dans lequel l'obtention de la pluralité d'éléments des données de capteur (18) à partir du système de capteur (26, 28, 30) comprend l'obtention de données, par un appareil de communication mobile (27), à partir du système de capteur (26, 28, 30) sur le réseau.

15. Produit de programme informatique comprenant des instructions de programme configurées pour programmer un processeur pour exécuter le procédé de l'une quelconque des revendications 1 à 14.
